# EUROPEAN PATENT SPECIFICATION

(11) **EP 2 921 135 B1**
(45) Date of publication and mention of the grant of the patent: **01.02.2017**
(21) Application number: 14160491.8
(22) Date of filing: 18.03.2014
(51) Int. Cl.: A61F 2/00, A61F 2/24, A61F 2/95

(54) **Prosthetic heart valve handling system**
Herzklappenprothesenhandhabungssystem
Système de manipulation de valvule cardiaque prothétique

(43) Date of publication of application: 23.09.2015
(73) Proprietor: NVT AG, 5630 Muri AG (CH)
(72) Inventor: Centola, Marcos, 72379 Hechingen (DE); Kawa, Emilia, 72379 Hechingen (DE)
(74) Representative: Witte, Weller & Partner Patentanwälte mbB

(56) References cited:
- US-A- 4 182 446
- US-A1- 2009 054 976
- US-A1- 2009 076 599

## Description

The present invention relates to a handling system for a prosthetic heart valve, in particular to a heart valve holder and a loading system.

### RELEVANT FIELD

Heart valve replacement is necessary where the native heart valve is damaged, mal- or nonfunctioning. For percutaneous replacement, various types and configurations of prosthetic heart valves are presently used, whereby the actual shape and configuration of any particular prosthetic heart valve is dependent, on the one hand, upon the valve being replaced. Generally, the prosthetic heart valve designs attempt to replicate the function of the valve being replaced and thus will regularly include valve leaflet-like structures used with either bioprostheses, which are usually made from animal tissues, either animal heart valve tissue or animal pericardial tissue, and which are treated to prevent rejection and to prevent calcification, or mechanical heart valve prostheses, which are generally composed entirely of synthetic or nonbiological materials. As such, the replacement valves may include a valved segment that is mounted in some manner within an (self-)expandable stent structure. There are two types of stents on which the valves structures are ordinarily mounted: self-expanding stents and balloon-expandable stents. To place such valves into a delivery apparatus and ultimately into a patient, the valve must first be collapsed or crimped to reduce its circumferential size.

For a fully functioning prosthetic heart valve it is crucial that all of its components fulfill their respective task: The valve, on the one hand, needs to be adequately attached to the stent support, since otherwise the valve is prone to failure, and valve failure, in the circulatory system, has significant consequences for the patient. On the other hand, the stent support needs to fully expand and, thus, guarantee the secure fixation within the heart vessels.

Accordingly, the handling and implantation of stented valve prostheses not only requires technical skill but also implies certain technical challenges, which are partly due to minimally-invasive procedures applied for implanting the prostheses and partly due to the delicate material used for replacement.

Prior to the implanting procedure, and in order to guarantee a smooth surgical intervention, the prosthetic heart valve, which has to be selected according to the patient's needs and conditions, has to be provided and stored in a state and by means guaranteeing its immediate and full function upon implantation. The stored prosthetic heart valve then needs to be taken from its container and loaded onto a deployment system, which needs to provide for an easy loading and handling itself. After loading onto the deployment system, the prosthetic heart valve is - via the deployment system - introduced into the patient's vessel system and moved forward to the heart valve to be replaced and subsequently deployed after reaching the correct position.

Thus, in particular with the delicate tissue-material of the biological valves it is mandatory to guarantee a secure handling and transporting of the valve, while at the same time preventing unnecessary stress to the prosthetic heart valve during storage. For a smooth implanting and replacement of the diseased heart valve it is also necessary to guarantee that the heart valve is correctly loaded.

Accordingly, there is a constant need for improving the handling systems and prosthetic heart valves that are currently available, facilitating the loading of the prosthetic valve onto the deployment system in order to securely deliver it to the valve that needs to be replaced, while simultaneously guaranteeing the smooth and easy release of the prosthetic heart valve in the heart vessel.

US 2009/054976 A1 discloses a prosthetic heart valve loading tool. The loading tool has a base and three prongs, extending therefrom for facilitating connection to the stent-mounted valve using notches found at the prong tips. Three additional short prongs extend from base between prongs.

Thus, it is an object of the present invention to provide for a handling system prosthetic heart valve that fulfills the requirements above and overcomes the drawbacks of the presently available prosthetic heart valves handling and loading systems.

### SUMMARY OF THE INVENTION

According the invention, this and other objects are solved by a prosthetic heart valve handling system for a prosthetic heart valve having a stent support and a valve structure comprising leaflets attached on the stent support, the heart valve handling system comprising a holder having a longitudinal axis extending from a proximal to a distal direction, the holder having a holding element, having a first end and a second end, first holder legs for stabilizing the holder, wherein the first holder legs are flexibly connected with the holding element's second end; first tongue elements for keeping open the leaflets of the prosthetic heart valve, wherein the first tongue elements are flexibly connected to the second end of the holding element; second tongue elements for fixating the prosthetic heart valve to the holder, wherein the second tongue elements are flexibly connected to the second end of the holding element, wherein the holder legs, the first tongue elements and the second tongue elements, respectively, are, at the second end of the holding element, flexible relative to the longitudinal axis of the holder, and wherein one of the holder legs, one of the first tongue elements and one of the second tongue elements, respectively, are coaxially arranged around a circumference of the holding element's second end.

The handling system according to the invention is particularly suited for securely holding a prosthetic heart valve during transportation and storage and for easily loading the prosthetic heart valve onto a deployment system adapted for the prosthetic heart valve and engaging with the handling system; thus, in the end, the prosthetic heart valve can be easily implanted while at the same time the full and immediate function of the prosthetic heart valve is guaranteed.

The handling system comprising a holder according to the invention, in connection and in cooperation with a loading means, a deployment system and a prosthetic heart valve, thus allows for an easy and precise handling, loading and deployment of the prosthesis: the prosthetic heart valve is sewed to the holder of the handling system; due to the particular shape of the handling system as defined in the attached claims, the valve can thus be fixed in the middle of, e.g., a jar or any other container containing a suitable liquid for storing the valve, thus preventing the valve to move with respect to the jar and touch its walls; the holder legs may be flexed around the stented valve prosthesis, thus protecting it from touching the jar walls, while at the same time the first tongue elements keep the leaflets open and the second tongue elements provide for attachment means of the the valve to the holder. Via the holding element, the holder may be grasped and placed into and withdrawn from the container.

As used herein, the expression "flexibly connected with" as used herein to describe the holder legs attachment with the holder is meant to designate that the legs are bendable relative to the holder or rather the holder's longitudinal axis: in the state where the prosthetic heart valve is not (yet) positioned into the holder the holder legs may be or are bend or angled, e.g. orthogonally angled, and, thus, spread outwardly relative to the longitudinal axis of the holder; in order to place or position the prosthetic heart valve within the holder, the legs may be or are inwardly bent towards the longitudinal axis of the holder, thus reducing the angle with which the legs are outwardly bent relative to the longitudinal axis of the holder. Thus, the legs have a first leg end by means of which they are attached to the holder/the holder's second end, and a second end which represents a free end. By bending the legs towards the longitudinal axis and away from the holding element, the holder as such can be placed on the free (second) end of the first holder legs, thus creating a space between them securely harboring the prosthetic heart valve.

As used herein, the term "proximal," when used in connection with a prosthetic heart valve, refers to the end of the heart valve closest to the heart when the heart valve is implanted in a patient, whereas the term "distal," when used in connection with a prosthetic heart valve, refers to the end of the heart valve farthest from the heart when the heart valve is implanted in a patient. Accordingly, when presently used, the term "distal direction" designates the direction towards the distal end of the prosthetic heart valve is pointing, and the "proximal direction" the direction towards the proximal end of the prosthetic heart valve is pointing.

According to a preferred embodiment, the holder further comprises second holder legs for guiding a prosthetic heart valve loading means there through, the second holder legs being shorter than the first holder legs, and being flexible relative to the longitudinal axis of the holder.

The second holder legs provide guiding means, preferably ate least one, preferably two, openings or holes by means of which a loading means or help can be guided through and loading of the prosthetic heart valve is facilitated.

The second holder legs each comprise a first end via which the second holder legs are flexibly connected to the holding element's second end, and a second end which may be flexibly bent to provide for a suitable angle of the second holder legs' second end.

According to a refinement of the heart valve handling system of the invention, the second holder legs are arranged around the circumference of the holding element's second end, such, that one second leg alternates with one first holder leg.

This embodiment ensures the stability of the holder as such as well as the stability of the loaded heart valve.

According to a refinement of the prosthetic heart valve handling system of the invention, it comprises three of each, first holder legs, second holder legs, first tongue elements and second tongue elements.

This embodiment advantageously reflects the number of leaflets of a valve, i.e. three, thus ensuring a stable positioning of the heart valve within the holder and a stable positioning of the holder as such in the state where the valve is held within the holder.

According to another refinement of the heart valve handling system of the invention, each of the second legs comprises a first end being connected with the holding element, and a second end having an opening through which a heart valve loading means can be guided.

According to this embodiment, the "opening" or "hole" in the second end of the second legs can have any form, i.e. oval, circular, rectangular, but is preferably adapted to the form of the loading means' parts that are to be guided through.

According to another embodiment, the second legs, further each comprise a second opening, which preferably extends over at least a portion of the second legs. In operation, the second legs can be bent or flexed towards the distal direction in general, and the second end of the second legs can, in turn, further be bent/flexed towards the longitudinal direction, such, that the opening at the end and the second opening get aligned.

According to another embodiment, or refinement of the heart valve handling system according to the invention, the holding element comprises a central tubular passage, and has, in another preferred embodiment, an outer diameter that has a substantially triangular shape.

This measure has the advantage that the holding element can be ergonomically and securely grasped and held by/between the fingers of the operator.

According to yet another embodiment of the heart valve handling system of the invention, each of the second tongue elements comprises an opening via which the valve can be fixated to the holder.

By fixating the valve to the holder, its secure holding within the holder is guaranteed. As soon as the patient is ready for implantation, the operator can grasp the holder by its holding element and withdraw the holder from the container the valve and the holder have been placed and/or stored and/or transported. Subsequently, the valve placed within the holder is, via a loading means, at least partially placed onto a deployment system. After loading, the holder is cut out, e.g. using scissors or a scalpel.

According to another aspect of the heart valve handling system of the invention, the first tongue elements are, respectively, cut-outs of the first holder legs, and according to yet another aspect, the second tongue elements are cut-outs of the first tongue elements.

In other words, the first holder legs and/or the first tongue elements have cut-outs the shape of which, respectively, correspond to the first tongue element's and the second tongue element's shape, respectively.

This measure as the advantage that the second tongue elements can be "integrated" within the first tongue elements, and are, thus, co-linearly "aligned" with respect to one another.

According to another embodiment of the heart valve handling system of the invention, the handling system further comprises a loading means, the loading means comprising (i) a conical funnel-shaped element extending from a first funnel opening to a second funnel opening, the first funnel opening being narrower than the second funnel opening, and (ii) bar-like longitudinal structures extending from a first end and to a second end, wherein the bar-like longitudinal structures, via their first end, are fixated to the funnel-shaped element, the second end extending away free relative to the funnel-shaped element.

This measure has the advantage that via the bar-like structures of the loading means, which as such represents a funnel-like element having bar-like extensions, and via the openings in the second holder leg, the loading means is engageable with the holder, i.e. at the end opposite of the holding element, such, that the heart valve is guided via the second funnel opening towards the first funnel opening; during this handling, the first holder legs can be bent again towards a more angled position with respect to the longitudinal axis of the holder. Subsequently, the operator can gently push the heart valve towards and into the second funnel opening thereby compressing it at this end; preferably the end of the prosthetic heart valve being compressed by the first funnel opening's smaller diameter/narrower opening, is then engageable from the outside of the funnel opening with a deployment system. E.g., the end of the prosthetic heart valve has, in its stent crown, engagement means, such as T-shaped endings in all of this free, preferably three, end which engage with a respective engaging means of the tip of deployment (catheter) system.

According to yet another refinement of the heart valve handling system according to the invention, the funnel element's second opening has a rim surrounding the second opening, wherein the bars are attached via their second end to the rim.

The invention also concerns the use of the handling system and to a method for loading a prosthetic heart valve on a deployment system. The method comprises according to the invention comprises the steps of:
a) providing (i) a handling system according to the invention, comprising a holder, a loading means, and a deployment system, the deployment system comprising a catheter and a sheath, and (ii) a prosthetic heart valve having a first end and a second send and comprising an expandable generally tubular stent support and a valve structure having a plurality of leaflets and a plurality of commissure points,
b) placing the prosthetic heart valve within the holder of the handling system and securing it to the holder,
c) introducing the prosthetic heart valve with its first end into the loading system thereby compressing its first end,
d) introducing the tip of the deployment system into the loading system and into the compressed first end of the prosthetic heart valve in order to fix the first end of the prosthetic heart valve to the deployment system and to partially load the prosthetic heart valve onto the catheter of the deployment system and covering the first end with the sheath of the deployment system,
e) cutting off the holder from the prosthetic heart valve, and
f) fully loading the prosthetic heart valve onto the catheter of the deployment system and covering it with the sheath.

According to a refinement of the method according to the invention, the method may comprise the step of rinsing and/ or cooling the prosthetic heart valve and/or the handling system according to the invention prior to loading the prosthetic heart valve onto the deployment system or while the prosthetic heart valve is partially and/or fully loaded onto the deployment system.

According to yet another refinement of the invention, step f) of the method according to the invention comprises the steps of (i) compressing the second end of the prosthetic heart valve and subsequently the whole prosthetic heart valve by stepwise introducing it into a counter-funnel element, and (ii) fully loading the prosthetic hart valve on the catheter of the deployment system and fully covering the prosthetic heart valve with the sheath.

With the method described herein, a precise and easy loading of the prosthetic heart valve onto its deployment system can be achieved. As a consequence, with the loaded prosthetic heart valve, the practitioner or surgeon or operator can carefully place and even re-place the prosthetic heart valve within a vessel.

It is understood that the features described hereinabove and those still to be described below fall within the scope of the present invention not only in the respectively specified combinations, but also in different combinations or on their own.

Further features follow from the description and the preferred embodiments.

Preferred embodiments are shown in the Figures and are described in further detail herein below.

### BRIEF DESCRIPTION OF THE FIGURES

In the figures:

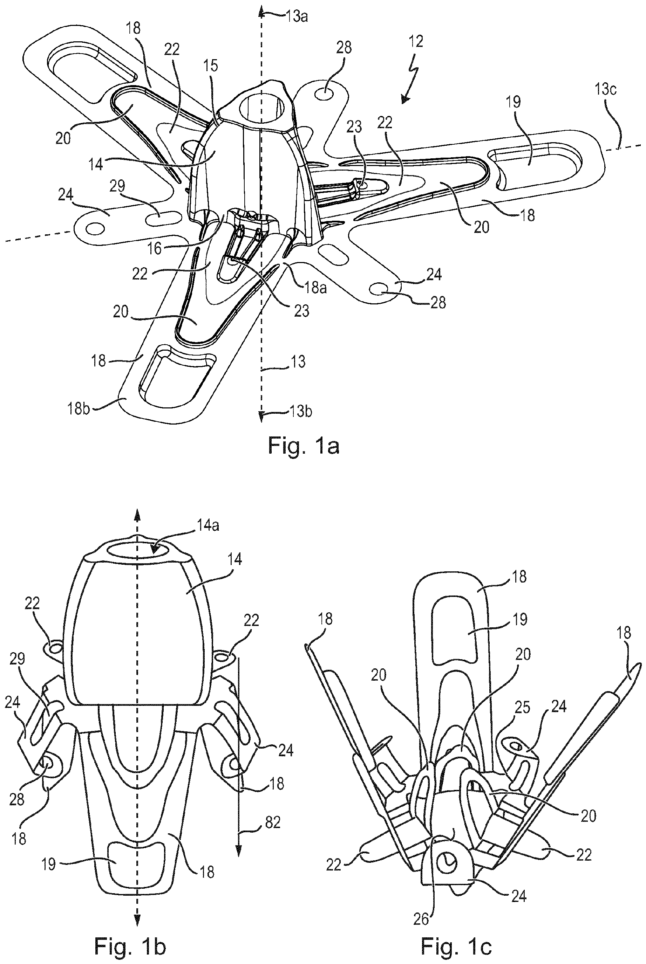

Fig. 1 shows a schematic drawing of an embodiment of the holder of the handling system according to the invention, where the first holder legs are perpendicular to the longitudinal axis of the holder (fig. 1a); and where the first holder legs are bent or flexed, shown in top view onto the holding element (fig. 1b) and in bottom view (fig. 1 c);

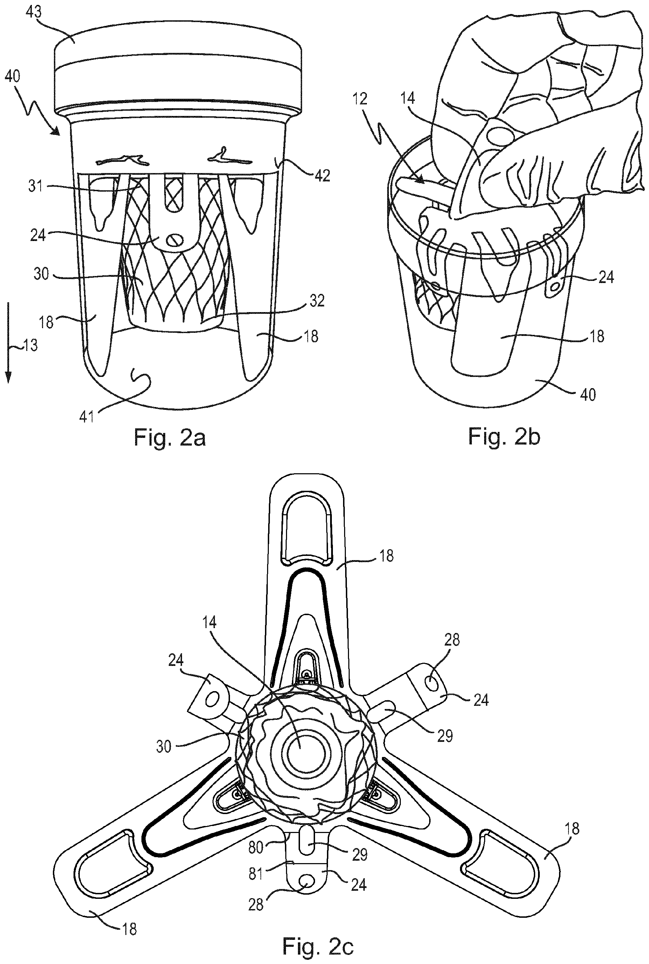

Fig. 2 shows a schematic drawing of the embodiment of the holder of fig. 1 having loaded thereon a prosthetic heart valve, with the holder comprising the prosthetic heart valve being placed in a container (fig. 2a), with the holder being grasped at the holding element and getting withdrawn from the container (fig. 2b), and with a bottom view of the holder comprising the prosthetic heart valve (fig. 2c);

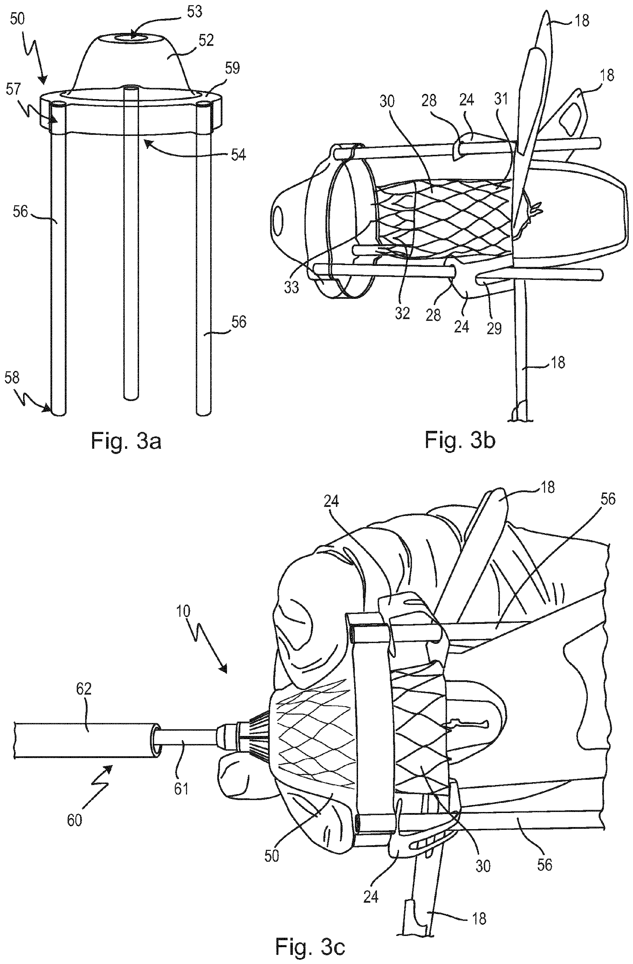

Fig. 3 shows a schematic drawing of an embodiment of the heart valve handling system with the handling system's loading means shown in detail in fig. 3a, with the loading means in engagement with the holder shown in fig. 3b, and a loading step during the method according to the invention, where the handling system's deployment system, the holder and the loading means are shown in engagement with one another (fig. 3c).

### DETAILLED DESCRIPTION OF THE FIGURES

Fig. 1 shows a schematic drawing of a holder 12 of the handling system 10 according to the invention. The holder 12 has a longitudinal axis 13 extending from a proximal direction 13a to a distal direction 13b; the holder 12 comprises a holding element 14 with a first end 15 and a second end 16. Further, the holder 12, at the holding element's 14 second end 16, comprises three first holder legs 18, which holder legs 18 are flexibly connected with the second end 16 of the holding element 14: The first legs 18 are bendable relative to the holder's longitudinal axis 13, such, that the they may be angled, e.g. orthogonally angled, and spread outwardly relative to the longitudinal axis 13 of the holder, towards a direction 13c that is orthogonally to the longitudinal axis 13. In fig. 1, the legs 18 are angled for about 90° relative to the longitudinal axis; however, they, i.e. all of the three first holder legs or only one or two, may be angled or bent in any other angle, whichever is convenient for storing, placing or handling of the holder 12, respectively. E.g., in case a prosthetic heart valve is loaded and to be stored, e.g. in a container filled with, e.g., a liquid, for later use, the fist holder legs 18 will be bent/angled towards the distal direction 13b in order to provide for a stable and protected cover for the prosthetic heart valve loaded on the holder 12. The position, where the first holder legs 18 are bent towards the distal direction 13b is shown in fig. 1 b.

In other words, the holder legs 18 have a first end 18a via which they are flexibly connected with the second end 16 of the holding element 14, and a free second end 18b, which represents the "foot", respectively, the holder is placed upon when the holder legs 18 are bent towards the distal direction 13b. As it is shown in fig. 1, the first holder legs comprise, within the region of the second end 18b, stabilizing pads 19.

The holder 12 further comprises three first tongue elements 20 for keeping open the leaflets of a prosthetic heart valve, as shown in fig. 1 c: The first tongue elements 20 can be fabricated as integrated parts of the first holder legs 18 and can be pushed or pressed out the form of the first holder legs 18 and towards the distal direction 13c as soon as a prosthetic heart valve is to be loaded on the holder 12. In other words, the first tongue elements 20 represents flap-like elements flexibly integrated within the first holder legs 18, that are, as the first holder legs 18 are, flexibly connected with the holder via the holding element's second end 16.

As can be seen in fig. 1a to 1 c, the holder 12 further comprises three second tongue elements 22, which are flexibly integrated within the first tongue elements 20 which in turn are flexibly integrated within the first holder legs 18, respectively. The second tongue elements 22 are intended for fixating a prosthetic heart valve to the holder 12, and accordingly comprise fixating elements, which in the embodiment shown in fig. 1 represent openings 23.

Via these openings 23, a prosthetic heart valve - once placed onto the holder 12 and once held open by the first tongue means 20 - can be fixated to the holder 12 via sutures; e.g., knots may be formed outside of the openings 23, i.e. on the side facing the outside of the holder 12.

The holding element 14 has an ergonomically formed outer shape, preferably a triangular diameter shape. In the inside, the holding element 14 preferably comprises a central tubular passage 14a.

As can also be taken from fig. 1, the holder 12 further comprises three second holder legs 24 for guiding a prosthetic heart valve loading means there through. The second holder legs 24, just like the first holder legs 18, are flexibly connected via their respective first end 25 of the second holder legs 24 to the holding element's 14 second end 16, and each of the second holder legs 24 has, respectively, a free second end 26, which free second end 26 carrying an opening 28 and which free second end 26 may be flexibly bent towards the longitudinal axis 13 in order to provide for a suitable angle of the second end 26.

The second holder legs 24, in the embodiment shown in fig. 1 to 3, further comprises a second opening 29 which preferably extends over at least a portion of the second leg 24, and, thus represents a longitudinal opening or window 29. This can also be taken from fig. 2a and 2c.

As can be seen from fig. 1, the first holder legs 18 and the second holder legs 24 are arranged around the circumference of the holding element's second end, such, that one first holder leg 18 alternates with a second holder leg respectively.

Fig. 2a shows a prosthetic heart valve 30 placed onto the holder 12 shown in fig. 1: In fig. 2a, the holder, together with the prosthetic heart valve 30 loaded thereon, is placed within a container 40. The prosthetic heart valve 30 has a first end 31 and a second end 32, wherein the prosthetic heart valve 30, via its first end 31 is attached, e.g. via sutures, to holder 12. The prosthetic heart valve 30 also comprises a generally cylindrical stent support 33 and a valve structure 34, wherein the valve structure 34 is attached to the interior of the stent support 33.

The container 40 has a cylindrical shape with a bottom 41 and a surrounding wall 42. The container may be closed with a lid 43 and can be filled with a liquid for storing the prosthetic heart valve 30 or a liquid in immediate preparation for getting implanted. The first holder legs 18 and second holder legs 24 are bent in the distal direction 13. Thus, in particular the holder legs 18 form a protective cage around the prosthetic heart valve 30, preventing the prosthetic heart valve 30 coming into contact with either bottom 41 or walls 42 of container 40.

The lid 43 can be taken off, and the holder 12 can be grasped by the operator or surgeon via the holding element 14, as indicated in fig. 2b. The prosthetic heart valve remains protected between the bent first holder legs 18, and can, e.g. be flushed or rinsed or cooled for further processing or loading procedures.

Fig. 2c shows a perspective view taken from the distal direction 13b towards the proximal direction 13a, displaying the prosthetic heart valve 30 placed onto the the holder 12. As indicated by the hypothetical lines 80 and 81 in fig. 2c, the second holder legs 24 may be bent/flexed at certain positions: The second holder legs 24 are, via their first end 25 flexibly, i.e., bendably connected with the second end 16 of the holding element 14 and may be bent at first line 80. The second end 26 of the second holder legs 24 can further be bent towards the longitudinal axis 13, at and along line 81 to bring opening 28 into virtual alignment with opening 29; via this alignment, a passage is generated for guiding means of a loading means as shown in fig. 3. The accordingly aligned openings 28, 29, formed by bending/flexing second holder leg 24 are also shown in fig. 1 c, where the thus formed passage is indicated by arrow 82 reaching through the openings 28, 29.

Fig. 3 shows in fig. 3a an embodiment of the handling system's 10 loading means 50. The loading means 50 comprises a conical funnel-shaped element 52 extending from a first funnel opening 53 to a second funnel opening 54. As can be seen from fig. 3a, the first funnel opening 53 is narrower than the second funnel opening 54, thus generating the funnel-shaped form of element 52.

The leading means 50 further comprises three bar-like longitudinal structures 56 having and extending from and between a first ends 57 and second ends 58. The second ends 58 of the longitudinal structures are engageable with the openings 28, 29 of the second holder legs 24 and are guided there through.

Via their first end 57, the longitudinal bar-like structures 56 are attached to a rim 59 circumferentially surrounding the second funnel opening 54 of funnel-shaped element 52.

Fig. 3b shows the engagement of the loading means 50 with the holder 12: the three bar-like longitudinal structures are guided through the openings or holes 28, 29 of the three second holder legs 24, with the wider second funnel opening facing towards the second end 32 of prosthetic heart valve 30. Thus, in operation and in order to load the prosthetic heart valve 30 onto a deployment system 60, as it is partially depicted in fig. 3c, the second end 32 of prosthetic heart valve 39 is moved towards and into the second funnel opening 54, where the prosthetic heart valve, with its second end 32, is further guided toward the narrower first funnel opening 53, whereby the second end 32 of prosthesis 30 gets compressed.

Fig. 3c shows the engagement of a deployment system 60, or rather of its catheter 61 carrying sheath 62, with the compressed second end 32 of prosthesis 30. The second end 32 of prosthetic heart valve 30 preferably carries T-bar-like endings (not shown) in its stented crown 36 engaging with respective engagement structures (not shown) in the tip of catheter 61, whereby the second end 32 of the prosthetic heart valve gets releaseably attached to the catheter's 61 tip. The sheath 62 can then be moved over the second end 32 of the prosthetic heart valve 30, e.g. by actuating respective actuating mechanisms of the deployment system 60, whereby the prosthetic heart valve gets further loaded onto the catheter 61 of deployment system 60.

The material of the holder 12 can be made of any plastics and is preferably transparent. The material of the loading means is preferably selected from plastics or metal, wherein it is preferred if the funnel-shaped element 52 is made from plastics and the bar-like longitudinal structures are mare from a metal.

In order to load the prosthetic heart valve 30 onto the catheter 61 of the deployment system 60, the following loading procedure or method or method steps can be performed:

A prosthetic heart valve 30 to replace the native valve of a patient is loaded onto a holder 12. The prosthetic heart valve 30 is, via its first end 31 secured, preferably sutured to the holder 12, preferably via the holder's 12 second tongue elements 22. The fixation can be achieved with knots of the sutures. The first tongue elements 20 keep the leaflets of the prosthetic heart valve 30 open. The arrangement of holder 12 and prosthetic heart valve can be stored and/or transported in a container 40.

Due to the holder's 12 structure the prosthetic heart valve 30 is placed and held in the middle of the container 40, with the stabilizing holder legs 18 preventing relative movements and preventing the prosthetic heart valve 30 from coming into contact with the walls 42 of container 40. Thus, the prosthetic heart valve 30 is securely protected and stored by holder 12 during storage and transportation. In addition, the first tongue elements 20 keep the leaflets of the prosthetic heart valve 30 open in order to guarantee full and immediate functioning of the prosthetic heart valve 30 after implantation. The container 40 is preferably filled with a liquid, the prosthetic heart valve 30, and, as a consequence, the holder 12 are stored in.

When the respective prosthetic heart valve 30 is to be implanted, lid 43 of container 40 is lifted off and holder 12 is grasped via the holding element 14 by the surgeon or operator. The prosthetic heart valve can now be rinsed prior to lading it onto the deployment system 60 and/or prior to using the loading means 50.

In order to load the prosthetic heart valve 30 onto the delivery system 60, the holder 12, via the openings 28, 29 of the second holder legs 24, presents guiding structures via which the bar-like-structures 56 of the loading means 50 can be guided and slide through. In that way, the prosthetic heart valve 30 can be orientated relative to the deployment/delivery system 60. The prosthetic heart valve 30 is, with its second end 32, gently pushed towards the second 54 and then the narrower first 53 funnel opening, thus compressing the second end 32 of the prosthetic heart valve 30. The three first tongue elements 20 of holder 12 still keep the leaflets of the prosthetic heart valve 30 open.

The deployment system's 60 catheter tip is then pushed through the compressed second end 32 of prosthetic heart valve 30, and the prosthetic heart valve's 30 second end 32 is engaged with engagement means in the tip of catheter 61, e.g. with connector grooves present in the tip of catheter 61. The sheath 62 may then be pushed over the second end 32 of the prosthetic heart valve 30, thereby covering it. The movement of the sheath may be, e.g., achieved by actuating a mechanism on the deployment system 60.

As soon as the valve portion of the prosthetic heart valve 30 is getting covered by the sheath, i.e. as soon as the prosthetic heart valve 30 is partially covered, the sutures of the holder 12 are cut and the holder 12 is removed, thus releasing the first end 31 of the prosthetic heart valve 30. At this stage, a further flushing or rinsing of the prosthetic heart valve 30 and /or the catheter 61 can be performed. Also, before fully loading of the prosthetic heart valve 30, it can be cooled in a ice-cold sterile 0.9% saline solution.

Next, the not-compressed and uncovered portion of the prosthetic heart valve 30 is introduced into a second funnel element, or counter funnel (not shown), thus gently compressing the remaining portion of the prosthetic heart valve 30; subsequently, the portion of the prosthetic heart valve 30 that has not been covered in the previous steps can be covered by the sheath 62 as far as only a few millimeters of the prosthetic heart valve 30 are exposed/uncovered.

According to a specific embodiment of the deployment system used according to the invention, the tip of the catheter 61 may comprise a tip funnel circumferentially surrounding the tip adjacent to the cover tip, which tip funnel is used to facilitate the introduction of the first end 31 of prosthetic heart valve 30 into the tip of catheter 61. The tip funnel may be removed and/or broken off as soon as the first end 31 of the prosthetic heart valve 30 is covered by the sheath, so as to fully introduce the very end of the first end 31 of prosthetic heart valve 30 into the cover tip.

As counter funnel or second funnel element, an ordinary funnel sized and configured to compress the first end 31 of prosthetic heart valve 30 can be used. It will be apparent to one skilled in the art which funnel should/can be used.

## Claims

1. Prosthetic Heart valve handling system (10) for a prosthetic heart valve (30) having a stent support (33) and a valve structure (34) comprising leaflets attached on the stent support (33), the prosthetic heart valve handling system (10) comprising:
a holder (12) having a longitudinal axis (13) extending from a proximal direction (13a) to a distal direction (13b), the holder (10) having
a holding element (14), having a first end (15) and a second end (16),
first holder legs (18) for stabilizing the holder (12), wherein the first holder legs (18) are flexibly connected with the holding element's (14) second end (16);
first tongue elements (20) for keeping open the leaflets of the prosthetic heart valve (30), wherein the first tongue elements (20) are flexibly connected to the second end (16) of the holding element (14);
second tongue elements (22) for fixating the prosthetic heart valve (30) to the holder (12), wherein the second tongue elements are flexibly connected to the second end (16) of the holding element (14),
wherein the holder legs (18), the first tongue elements (20) and the second tongue elements(22), respectively, are, at the second end (16) of the holding element (14), flexible relative to the longitudinal axis (13) of the holder (12), and wherein, each, one of the holder legs (18), one of the first tongue elements (20) and one of the second tongue elements (22), respectively, are coaxially arranged around a circumference of the holding element's (14) second end (16).

2. The prosthetic heart valve handling system (10) of claim 1, wherein the holder (12) further comprises
second holder legs (24) comprising at least one, preferably two, opening (28, 29) for guiding a prosthetic heart valve loading means (50) there through, the second holder legs (24) being shorter than the first holder legs (18), and being flexible relative to the longitudinal axis (13) of the holder (12).

3. The prosthetic heart valve handling system (10) of claim 2, wherein the first holder legs(18) and second holder legs (24) are arranged around the circumference of the holding element's (14) second end (16), such, that one second holder leg (24) alternates with one first holder leg (18).

4. The prosthetic heart valve handling system (10) of any of claims 1 to 3, comprising three of each, first holder legs (18), second holder legs (24), first tongue elements (20) and second tongue elements (22).

5. The prosthetic heart valve handling system of any of claims 2 to 4, wherein each of the second legs comprises a first end being connected with the holding element, and a second end having an opening through which a heart valve loading means can be guided.

6. The prosthetic heart valve handling system (10) of any of claims 1 to 5, wherein the holding element (14) comprises a central tubular passage (14a).

7. The prosthetic heart valve handling system (10) of any of claims 1 to 6, wherein the holding element (14) has a diameter that is substantially triangular.

8. The prosthetic heart valve handling system (10) of any of claims 1 to 7, wherein each of the second tongue elements (22) comprises an opening (23) via which the prosthetic heart valve (30) can be fixated to the holder (12).

9. The prosthetic heart valve handling system (10) of any of claims 1 to 8, wherein the first tongue elements (20) are cut-outs of the first holder legs (18).

10. The prosthetic heart valve handling system (10) of any of claims 1 to 9, wherein the second tongue elements (22) are cut-outs of the first tongue elements (20).

11. The prosthetic heart valve handling system (10) of any of claims 2 to 10 further comprising a loading means (50), the loading means comprising (i) a conical funnel-shaped element (52) extending from a first funnel opening (53) to a second funnel opening (54), the first funnel opening (53) being narrower than the second funnel opening (54), and (ii) bar-like longitudinal structures (56) extending from a first end (57) and to a second end (58), wherein the bar-like longitudinal structures (56), via their first end (57), are fixated to the funnel-shaped element (52), the second end (58) extending away free relative to the funnel-shaped element (52).

12. The prosthetic heart valve handling system (10) of claim 11, wherein the bar-like longitudinal structures (56), via their free second end (58) are engageable with the second holder legs' (24) openings (28, 29) present in a second end (26) of second the holder legs (24).

13. The prosthetic heart valve handling system (10) of claim 11 or 12, wherein the funnel element's second opening (54) has a rim (59) surrounding the second opening (54), wherein the bar-like structures (56) are attached via their first end (57) to the rim (59).

14. The prosthetic heart valve handling system (10) according to any of claims 1 to 13, further comprising a prosthetic heart valve (30), the prosthetic heart valve (30) comprising an expandable generally tubular stent support (33) and a valve structure (34).

15. The prosthetic heart valve handling system (10) according to any of claims 1 to 14, further comprising a deployment system (60).

16. Use of a prosthetic heart valve handling system (10) according to any of claims 1 to 15 for loading a prosthetic heart valve (30) on a prosthetic heart valve deployment system (60).

17. Method for loading a prosthetic heart valve (30) onto a prosthetic heart valve deployment system (60), comprising the steps of
a) providing (i) a prosthetic heart valve handling system (10) according any of claims 1 to 15, comprising a holder (12), at least one loading means (50), and a deployment system (60 comprising a catheter 61, a sheath (62), and a catheter tip, and (ii) a prosthetic heart valve (30) having a first end (31) and a second end (32) and comprising an expandable generally tubular stent support (33) and a valve structure (34) having a plurality of leaflets and a plurality of commissure points,
b) placing the prosthetic heart valve (30) within the holder (12) of the handling system (10) and securing it to the holder (12),
c) introducing the prosthetic heart valve (30) with its first end (31) into the loading system (50) thereby compressing its first end (31),
d) introducing the tip of the deployment system (60) into the loading system (50) and into the compressed first end (31) of the prosthetic heart valve (30) in order to fix the first end (31) of the prosthetic heart valve (30) to the deployment system (60) and to partially load the prosthetic heart valve (30) onto the catheter (61) of the deployment system (60) and covering the first end (31) with the sheath (62) of the deployment system (60),
e) cutting off the holder (12) from the prosthetic heart valve (30), and
f) fully loading the prosthetic heart valve (30) onto the catheter (61) of the deployment system (60) and covering it with the sheath (62).

## Patentansprüche

1. Herzklappenprothesen-Handhabungssystem (10) für eine Herzklappenprothese (30), die einen Stent-Träger (33) sowie eine Klappenstruktur (33) mit an den Stent-Träger (33) angebrachten Segeln aufweist, wobei das Herzklappenprothesen-Handhabungssystem (10) Folgendes aufweist:
einen Halter (12) mit einer Längsachse (13), die sich von einer proximalen Richtung (13a) in eine distale Richtung (13b) erstreckt, wobei der Halter (10)
ein Halteelement (14) mit einem ersten Ende (15) und einem zweiten Ende (16) aufweist; ferner
erste Halter-Beinchen (18) zur Stabilisierung des Halters (12), wobei die ersten Halter-Beinchen (18) mit dem zweiten Ende (16) des Halteelements (14) flexibel verbunden sind;
erste Laschenelemente (20) zur Offenhaltung der Segel der Herzklappenprothese (30), wobei die ersten Laschenelemente (20) mit dem zweiten Ende (16) des Halteelements (14) flexibel verbunden sind;
zweite Laschenelemente (22) zur Fixierung der Herzklappenprothese (30) an den Halter (12), wobei die zweiten Laschenelemente (22) mit dem zweiten Ende (16) des Halteelements (14) flexibel verbunden sind;
wobei die Halter-Beinchen (18), die ersten Laschenelemente (20) und die zweiten Laschenelemente (22) jeweils am zweiten Ende (16) des Halteelements (14) in Bezug auf die Längsachse (13) des Halters (12) flexibel sind, und wobei jeweils eines der Halter-Beinchen (18), eines der ersten Laschenelemente (20) und eines der zweiter Laschenelemente (22) um einen Umfang des zweiten Endes (16) des Halteelements (14) koaxial angeordnet sind.

2. Herzklappenprothesen-Handhabungssystem (10) nach Anspruch 1, wobei der Halter (12) ferner folgendes aufweist:
zweite Halter-Beinchen (24) mit zumindest einem, vorzugsweise zwei Öffnungen (28, 29) für das Hindurchführen von Mitteln (50) zum Laden einer Herzklappenprothese, wobei die zweiten Halter-Beinchen (24) kürzer sind als die ersten Halter-Beinchen (18) und relativ zur Längsachse (13) des Halters (12) flexibel sind.

3. Herzklappenprothesen-Handhabungssystem (10) nach Anspruch 2, wobei die ersten Halter-Beinchen (18) und die zweiten Halter-Beinchen (24) um den Umfang des zweiten Endes (16) des Halteelements (14) herum angeordnet sind, und zwar derart, dass sich ein zweites Halter-Beinchen (24) mit einem ersten Halter-Beinchen (18) abwechselt.

4. Herzklappenprothesen-Handhabungssystem (10) nach einem der Ansprüche 1 bis 3, wobei das Herzklappenprothesen-Handhabungssystem (10) jeweils 3 von den ersten Halter-Beinchen (18), den zweiten Halter-Beinchen (24), den ersten Laschenelementen (20) und den zweiten Laschenelementen (22) aufweist.

5. Herzklappenprothesen-Handhabungssystem (10) nach einem der Ansprüche 2 bis 4, wobei jedes der zweiten Halter-Beinchen (24) ein erstes Ende (25) aufweist, das mit dem Halteelement (14) verbunden ist, sowie ein zweites Ende (26) mit einer Öffnung (28), durch welche hindurch Mittel (50) zum Laden einer Herzklappenprothese (30) geführt werden können.

6. Herzklappenprothesen-Handhabungssystem (10) nach einem der Ansprüche 1 bis 5, wobei das Halteelement (14) eine zentrale röhrchenförmige Passage (14a) aufweist.

7. Herzklappenprothesen-Handhabungssystem (10) nach einem der Ansprüche 1 bis 6, wobei das Halteelement (14) einen im Wesentlichen dreieckigen Durchmesser aufweist.

8. Herzklappenprothesen-Handhabungssystem (10) nach einem der Ansprüche 1 bis 7, wobei jedes der zweiten Laschenelemente (22) eine Öffnung (23) aufweist, über welche die Herzklappenprothese (30) an den Halter fixiert werden kann.

9. Herzklappenprothesen-Handhabungssystem (10) nach einem der Ansprüche 1 bis 8, wobei die ersten Laschenelemente (20) Ausschnitte der ersten Halter-Beinchen (18) sind.

10. Herzklappenprothesen-Handhabungssystem (10) nach einem der Ansprüche 1 bis 9, wobei die zweiten Laschenelemente (22) Ausschnitte der ersten Laschenelemente (20) sind.

11. Herzklappenprothesen-Handhabungssystem (10) nach einem der Ansprüche 2 bis 10, welches ferner Mittel zum Laden (50) aufweist, wobei die Mittel zum Laden (i) ein konisches, Trichter-förmiges Element (52) aufweisen, das sich von einer ersten Trichter-Öffnung (53) zu einer zweiten Trichter-Öffnung (54) erstreckt, wobei die erste Trichter-Öffnung (53) enger als die zweite Trichter-Öffnung (54) ist, sowie (ii) stäbchenförmige längliche Strukturen (56), die sich von einem ersten Ende (57) und einem zweiten Ende (58) erstrecken, wobei die stäbchenförmigen längliche Strukturen (56) über ihr erstes Ende (57) an das Trichter-förmige Element (52) fixiert sind, wobei sich das zweite Ende (58) in Bezug auf das Trichter-förmige Element (52) frei wegerstrecken kann.

12. Herzklappenprothesen-Handhabungssystem (10) nach Anspruch 11, wobei die stäbchenförmigen längliche Strukturen (56) über ihr freies zweite Ende (58) mit den Öffnungen (28, 29) der zweiten Halter-Beinchen (24) zusammenwirken, welche Öffnungen (28, 29) an einem zweiten Ende (26) der zweiten Halter-Beinchen (24) vorliegen.

13. Herzklappenprothesen-Handhabungssystem (10) nach einem der Ansprüche 11 oder 12, wobei die zweite Öffnung (54) des Trichter-Elements einen Rand (59) aufweist, der die zweite Öffnung (54) umgibt, wobei die stäbchen-förmigen Strukturen (56) über deren erstes Ende (57) an dem Rand (24) angebracht sind.

14. Herzklappenprothesen-Handhabungssystem (10) nach einem der Ansprüche 1 bis 13, das ferner eine Herzklappenprothese (30) aufweist, wobei die Herzklappenprothese (30) einen expandierbaren, im Wesentlichen röhrchenförmigen Stent-Träger (33) sowie eine Klappenstruktur (34) aufweist.

15. Herzklappenprothesen-Handhabungssystem (10) nach einem der Ansprüche 1 bis 14, das ferner ein Freisetzungssystem (60) aufweist.

16. Verwendung eines Herzklappenprothesen-Handhabungssystems (10) nach einem der Ansprüche 1 bis 15, zur Ladung einer Herzklappenprothese (30) auf ein Herzklappenprothesen-Freisetzungssystem (60).

17. Verfahren zur Ladung einer Herzklappenprothese (30) auf ein Herzklappenprothesen-Freisetzungssystem (60), welches die folgenden Schritte aufweist:
a) Bereitstellen von (i) einem Herzklappenprothesen-Handhabungssystem (10) nach einem der Ansprüche 1 bis 15, mit einem Halter (12), zumindest einem Mittel zum Laden (50), sowie einem Freisetzungssystems (60), das einen Katheter (61), eine Hülle (62), und eine Katheterspitze aufweist, und (ii) einer Herzklappenprothese (3) mit einem ersten Ende (31) und einem zweiten Ende (32), und mit einem expandierbaren, im Wesentlichen röhrchenförmigen Stent-Träger (33) sowie einer Klappenstruktur (34) mit einer Vielzahl an Segeln und einer Vielzahl an Kommisurpunkten,
b) Platzieren der Herzklappenprothese (30) innerhalb des Halters (12) des Handhabungssystems (10) und Befestigen der Herzklappenprothese (30) an den Halter (12),
c) Einführen der Herzklappenprothese (30) mit deren ersten Ende (31) in das Ladesystem (50), wodurch deren erstes Ende (31) komprimiert wird,
d) Einführen der Spitze des Freisetzungssystems (60) in das Ladesystem (50) und in das komprimierte erste Ende (31) der Herzklappenprothese (30), um das erste Ende (31) der Herzklappenprothese (30) an das Freisetzungssystem (60) zu fixieren und um die Herzklappenprothese (30) teilweise auf den Katheter (61) des Freisetzungssystems (60) zu laden, und Überdecken des ersten Endes (31) mit der Hülle (62) des Freisetzungssystems (60),
e) Trennen des Halters (12) von der Herzklappenprothese (30), und
f) vollständiges Laden der Herzklappenprothese (30) auf den Katheter (61) des Freisetzungssystems (60) und Überdecken der Herzklappenprothese mit der Hülle (62).

## Revendications

1. Système (10) de manipulation de valvule cardiaque prothétique destiné à une valvule (30) cardiaque prothétique muni d'un support (33) de tuteur et d'une structure (34) de valvule comportant des feuillets fixés sur le support (33) de tuteur, le système (10) de manipulation de valvule cardiaque prothétique comportant :
un dispositif de maintien (12) présentant un axe (13) longitudinal qui s'étend d'une direction (13a) proximale à une direction (13b) distale, le dispositif de maintien (10) présentant
un élément (14) de maintien, présentant une première extrémité (15) et une deuxième extrémité (16),
des premières branches (18) de dispositif de maintien permettant de stabiliser le dispositif de maintien (12), dans lequel les premières branches (18) de dispositif de maintien sont reliées de manière souple à la deuxième extrémité (16) de l'élément (14) de maintien ;
des premiers éléments (20) languette permettant de maintenir les feuillets de la valvule (30) cardiaque prothétique ouverts, les premiers éléments (20) languette étant reliés de manière souple à la deuxième extrémité (16) de l'élément (14) de maintien ;
des deuxièmes éléments (22) languette permettant de fixer la valvule (30) cardiaque prothétique sur le dispositif de maintien (12), lesdits deuxièmes éléments languette étant reliés de manière souple à la deuxième extrémité (16) de l'élément (14) de maintien,
dans lequel les branches (18) de dispositif de maintien, les premiers éléments (20) languette et les deuxièmes éléments (22) languette respectifs, sont, au niveau de la deuxième extrémité (16) de l'élément (14) de maintien, souples par rapport à l'axe (13) longitudinal du dispositif de maintien (12), et dans lequel chacun parmi l'une des branches (18) de dispositif de maintien, l'un des premiers éléments (20) languette et l'un des deuxièmes éléments (22) languette respectifs, sont agencés coaxialement autour d'une circonférence de la deuxième extrémité (16) de l'élément (14) de maintien.

2. Système (10) de manipulation de valvule cardiaque prothétique selon la revendication 1, dans lequel le dispositif de maintien (12) comporte en outre
des deuxièmes branches (24) de dispositif de maintien comportant au moins une, de préférence deux, ouvertures (28, 29) permettant de guider un moyen (50) de chargement de valvule cardiaque prothétique à travers celles-ci, les deuxièmes branches (24) de dispositif de maintien étant plus courtes que les premières branches (18) de dispositif de maintien, et étant souples par rapport à l'axe (13) longitudinal du dispositif de maintien (12).

3. Système (10) de manipulation de valvule cardiaque prothétique selon la revendication 2, dans lequel les premières branches (18) de dispositif de maintien et les deuxièmes branches (24) de dispositif de maintien sont agencées autour de la circonférence de la deuxième extrémité (16) de l'élément (14) de maintien, de telle sorte qu'une deuxième branche (24) de dispositif de maintien alterne avec une première branche (18) de dispositif de maintien.

4. Système (10) de manipulation de valvule cardiaque prothétique selon l'une quelconque des revendications 1 à 3, comportant trois de chacun parmi les premières branches (18) de dispositif de maintien, les deuxièmes branches (24) de dispositif de maintien, les premiers éléments (20) languette et les deuxièmes éléments (22) languette.

5. Système (10) de manipulation de valvule cardiaque prothétique selon l'une quelconque des revendications 2 à 4, dans lequel chacune des deuxièmes branches (24) de dispositif de maintien comporte une première extrémité (26) qui est reliée à l'élément de maintien (14), et une deuxième extrémité (26) présentant une ouverture (28) à travers laquelle le moyen (50) de chargement de valvule cardiaque (30) peut être guidé.

6. Système (10) de manipulation de valvule cardiaque prothétique selon l'une quelconque des revendications 1 à 5, dans lequel l'élément (14) de maintien comporte un passage (14a) central tubulaire.

7. Système (10) de manipulation de valvule cardiaque prothétique selon l'une quelconque des revendications 1 à 6, dans lequel l'élément (14) de maintien présente un diamètre qui est sensiblement triangulaire.

8. Système (10) de manipulation de valvule cardiaque prothétique selon l'une quelconque des revendications 1 à 7, dans lequel chacun des deuxièmes éléments (22) languette comporte une ouverture (23) par le biais de laquelle la valvule (30) cardiaque prothétique peut être fixée au dispositif de maintien (12).

9. Système (10) de manipulation de valvule cardiaque prothétique selon l'une quelconque des revendications 1 à 8, dans lequel les premiers éléments (20) languette sont des découpes des premières branches (18) de dispositif de maintien.

10. Système (10) de manipulation de valvule cardiaque prothétique selon l'une quelconque des revendications 1 à 9, dans lequel les deuxièmes éléments (22) languette sont des découpes des premiers éléments (20) languette.

11. Système (10) de manipulation de valvule cardiaque prothétique selon l'une quelconque des revendications 2 à 10 comportant en outre un moyen (50) de chargement, le moyen de chargement comportant (i) un élément (52) conique en forme d'entonnoir qui s'étend d'une première ouverture (53) d'entonnoir à une deuxième ouverture (54) d'entonnoir, la première ouverture (53) d'entonnoir étant plus étroite que la deuxième ouverture (54) d'entonnoir, et (ii) des structures (56) longitudinales semblables à des barres qui s'étendent d'une première extrémité (57) à une deuxième extrémité (58), dans lequel les structures (56) longitudinales semblables à des barres, par le biais de leur première extrémité (57), sont fixées à l'élément (52) en forme d'entonnoir, la deuxième extrémité (58) s'étendant libre, à distance, par rapport à l'élément (52) en forme d'entonnoir.

12. Système (10) de manipulation de valvule cardiaque prothétique selon la revendication 11, dans lequel les structures (56) longitudinales semblables à des barres, par le biais de leur deuxième extrémité (58) libre peuvent être mises en prise avec les ouvertures (28, 29) des deuxièmes branches (24) de dispositif de maintien présentes dans une deuxième extrémité (26) des deuxièmes branches (24) de dispositif de maintien.

13. Système (10) de manipulation de valvule cardiaque prothétique selon la revendication 11 ou 12, dans lequel la deuxième ouverture (54) de l'élément en entonnoir présente un rebord (59) entourant la deuxième ouverture (54), les structures (56) semblables à des barres étant fixées par le biais de leur première extrémité (57) au rebord (59).

14. Système (10) de manipulation de valvule cardiaque prothétique selon l'une quelconque des revendications 1 à 13, comportant en outre une valvule (30) cardiaque prothétique, la valvule (30) cardiaque prothétique comportant un support (33) de tuteur expansible généralement tubulaire et une structure (34) de valvule.

15. Système (10) de manipulation de valvule cardiaque prothétique selon l'une quelconque des revendications 1 à 14, comportant en outre un système (60) de déploiement.

16. Utilisation d'un système (10) de manipulation de valvule cardiaque prothétique selon l'une quelconque des revendications 1 à 15 permettant de charger une valvule (30) cardiaque prothétique sur un système (60) de déploiement de valvule cardiaque prothétique.

17. Procédé permettant de charger une valvule (30) cardiaque prothétique sur un système (60) de déploiement de valvule cardiaque prothétique, comportant les étapes consistant à
a) fournir (i) un système (10) de manipulation de valvule cardiaque prothétique selon l'une quelconque des revendications 1 à 15, comportant un dispositif de maintien (12), au moins un moyen (50) de chargement, et un système (60) de déploiement comportant un cathéter 61, une gaine (62), et un embout de cathéter, et (ii) une valvule (30) cardiaque prothétique présentant une première extrémité (31) et une deuxième extrémité (32) et comportant un support (33) de tuteur expansible généralement tubulaire et une structure (34) de valvule présentant une pluralité de feuillets et une pluralité de points de commissures,
b) placer la valvule (30) cardiaque prothétique à l'intérieur du dispositif de maintien (12) du système (10) de manipulation et l'immobiliser sur le dispositif de maintien (12),
c) introduire la valvule (30) cardiaque prothétique avec sa première extrémité (31) dans le système (50) de chargement, comprimant ainsi sa première extrémité (31),
d) introduire l'embout du système (60) de déploiement dans le système (50) de chargement et dans la première extrémité (31) comprimée de la valvule (30) cardiaque prothétique afin de fixer la première extrémité (31) de la valvule (30) cardiaque prothétique sur le système (60) de déploiement et de charger partiellement la valvule (30) cardiaque prothétique sur le cathéter (61) du système (60) de déploiement et recouvrir la première extrémité (31) avec la gaine (62) du système (60) de déploiement,
e) séparer le dispositif de maintien (12) de la valvule (30) cardiaque prothétique, et
f) charger entièrement la valvule (30) cardiaque prothétique sur le cathéter (61) du système (60) de déploiement et la recouvrir avec la gaine (62).
